(19) Europäisches Patentamt
European Patent Office
Office européen des brevets

(11) Publication number: **0 029 143**
**B1**

(12) **EUROPEAN PATENT SPECIFICATION**

(45) Date of publication of patent specification: **21.12.83**

(51) Int. Cl.³: **C 07 C 17/156,**
**C 07 C 19/045**

(21) Application number: **80106660.6**

(22) Date of filing: **30.10.80**

(54) **Catalyst composition and copper-catalyzed fluid-bed hydrocarbon-oxyhydrochlorination process for the preparation of 1,2-dichlorethane.**

(30) Priority: **05.11.79 US 91289**

(43) Date of publication of application:
**27.05.81 Bulletin 81/21**

(45) Publication of the grant of the patent:
**21.12.83 Bulletin 83/51**

(84) Designated Contracting States:
**DE FR GB NL**

(56) References cited:
**US - A - 3 657 367**
**US - A - 4 123 389**

(73) Proprietor: **The B.F. GOODRICH Company**
**Dept. 0015 WHB-6 500 South Main Street**
**Akron, Ohio 44318 (US)**

(72) Inventor: **Cowfer, Joseph Allen**
**1115 Gentry Drive**
**Medina Ohio 44256 (US)**
Inventor: **Eden, Jamal Shahab**
**502 North Revere Road**
**Akron Ohio 44313 (US)**
Inventor: **Magistro, Angelo Joseph**
**11017 Brookview Drive**
**Brecksville Ohio 44141 (US)**

(74) Representative: **von Kreisler, Alek, Dipl.-Chem. et al,**
**Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 029 143 B1

# Catalyst composition and copper-catalyzed fluid-bed hydrocarbon-oxyhydrochlorination process for the preparation of 1,2-dichlorethane

## Background of the invention

This invention pertains to fluid bed catalytic oxyhydrochlorination of hydrocarbons, particularly ethylene, to produce chlorinated hydrocarbons, particularly 1,2-dichloroethane, commonly called ethylene dichloride (EDC). It relates specifically to a novel method, and a composition of matter, for improving the fluidized copper catalyst used in such oxyhydrochlorination.

EDC is most easily produced commercially by the direct chlorination of ethylene, and is used in greatest quantity for pyrolysis, or "cracking", to produce vinyl chloride monomer (VCM), on which the vinyl plastic industry depends. The pyrolysis reaction produces, in addition to VCM, by-product hydrogen chloride (HCl) which is advantageously utilized at the plant site to produce more EDC for the pyrolysis. This is accomplished by the process called ethylene oxyhydrochlorination (or sometimes, more simply "oxychlorination") which involves the reaction of HCl with oxygen, supplied as such or as air, and ethylene in accordance with the empirical equation:

$$2\ C_2H_4+O_2+4\ HCl \rightarrow 2CH_2ClCH_2Cl+2H_2O$$

The ethylene oxychlorination is carried out in many highly successful commercial installations through the world by passing the gaseous reactants at elevated temperature and pressure through a fluidized solid catalyst bed in the manner and under the conditions generally described in Harpring et al U.S. patent 3,488,398. As taught by the Harpring et al patent, in order to achieve efficient utilization of HCl for formation of EDC, the molar ratio of ethylene to oxygen to HCl is to be maintained in the range of 1.0 to 1.2 moles ethylene to 0.55 to 0.9 moles oxygen for each two moles of HCl, with the most preferred ratio of ethylene to oxygen to HCl about 1.0 to 0.8/2.0. The process is operated at a temperature in the range of 190° to 250°C, preferably under a pressure of 10—50 psig.

The nature of the fluidized catalyst bed is of importance to the success of the ethylene oxyhydrochlorination process. The catalyst bed consists essentially of a copper compound, preferably copper chloride, uniformly distributed or carried on a fluidizable support, which is a particulate material of the proper ratio of particle sizes, surface area, porosity, density, resistant to attrition and other characteristics to provide proper fluidization and isothermal conditions in the reactor bed, to permit adequate contact between the copper catalyst and the gaseous reactants as they pass through the bed, and to minimize loss of catalyst through passage of fine particles from the reactor with the effluent gases. The fluidizable support can be silica, kieselguhr, fuller's earth, various clays, alumina or the like. The preferred support, as taught by the Harpring et al patent, is composed of alumina, most desirably activated alumina or microgel alumina since such supports exhibit superior resistance to attrition and ability to fluidize and can be readily prepared to have the desired surface area and ratio of particle sizes in accordance with the "Bayer process" or other bauxite calcination techniques well known to the art. Preparation of the copper catalyst on the fluidizable support is well known to the art, and is described in the referenced Harpring et al patent. Typically the copper compound is dissolved in water, and the solution is slowly sprayed on the support with continuous mixing (or alternatively adding the support to the solution with mixing) followed by drying the wet subject until it is free flowing, calcining for a few hours at a temperature of 110°C, and screening to eliminate large particles. The supported catalyst is then ready for addition to the oxyhydrochlorination reactor to function as the fluidized catalyst bed. The supported catalyst is prepared to contain from 2 to 10 percent by weight copper. It is customarily supplied to the operator of the ethylene oxyhydrochlorination process for addition to the reactor at "start-up" of the process or as "make-up" when the catalyst bed needs replenishing.

Copper catalysts prepared as above have serviced well as oxyhydrochlorination catalysts. However, all such catalysts exhibit, to a more or less degree, a tendency to agglomerate, a characteristic which is called "stickiness" in the trade. The degree of stickiness of the catalyst is dependent on many factors, including the pressure and temperature of the reaction, the absorptive nature or porosity of the catalyst, the amount and distribution of the copper on the particle surfaces, the ratio of the weight of copper to the surface area of the support, the number of active sites available on the catalyst and the manner and degree of their utilization, the presence of contaminants such as sulfur, as well as upon the quantity and ratios of the gaseous reactants in the fluid bed. A certain degree of stickiness is tolerable, but if the catalyst is so sticky that particles continually agglomerate and are not broken up by movement in the fluidized bed, "hot spots" are developed in the bed at the point of the agglomeration, especially at the bottom of the bed. These hot spots may eventually lead to loss of fluidity or "inversion" and total collapse of the fluidized bed. Even if inversion does not occur, agglomeration of the catalyst can cause plugging of the lower portion or "dip leg" of the cyclone above the reactor (indicated by reference numeral 14 of figure 1a of the drawing of Harpring et al U.S. patent 3,488,398 which cyclines separate the catalyst fines from the effluent gases and retain the catalyst in the bed) with the result that large quantities of the catalyst can be lost and operations disrupted. To a large extent, the stickiness of the copper catalyst can be controlled by efficient operation of the process. In US Patent 3 657 367 a process is described for the oxychlorination of hydrocarbons with non-caking

catalysts which however must contain didymium, lanthanum and/or magnesium on a support and which require reaction temperatures of 300—600°C. An improved catalyst for oxyhydrochlorination with reduced melting-properties is described in US-Patent 4 123 389. However, a more effective and practical way to inhibit and/or reduce stickiness of the catalyst during operation would be very desirable.

Summary of the invention

According to the instant invention a process is described for the oxyhydrochlorination of a gaseous hydrocarbon containing 1 to 4 carbon atoms to form a chlorinated hydrocarbon, in which the hydrocarbon, an oxygen containing gas and gaseous hydrogen chloride are passed at a temperature of 190° to 250°C through a fluidized bed of a catalyst containing a copper compound deposited on a fluidizable support, characterized in that at least a substantial portion of the copper compound is deposited on at least a substantial portion of the fluidizable support in situ in the fluidized bed, thereby preventing particles of supported catalyst from sticking to one another in the fluid bed and adversely affecting its fluidization properties.

This invention prevents or substantially inhibits or alleviates stickiness in fluid bed catalysts composed of copper compound on a fluidizable support, when used in hydrocarbon oxyhydrochlorination processes, particularly ethylene oxyhydrochlorination to produce EDC. However, this invention can also be used in the oxyhydrochlorination of other aliphatic hydrocarbons containing 1 to 4 carbon atoms such as methane and ethane to produce chlorinated hydrocarbons such as chloroform, carbon tetrachloride, trichloroethane, and the like. The invention accomplishes this through the expedient of depositing at least a substantial portion of the copper compound on the fluidizable support while the latter is in the fluidized condition, i.e. with the support particles in movement suspended by a flow of gases at the temperature of the reaction. Hence, instead of preparing the supported copper catalyst for the fluid bed entirely outside the reactor, as in the prior practice, at least a substantial portion of it is prepared in situ.

The in situ catalyst preparation is accomplished conveniently in any of several ways through use of bare fluidizable support, that is to say, a support, especially alumina, of the requisite particle size distribution, surface area and other characteristics, on which no copper compound is deposited. The bare support can be used in substantial proportion with support on which copper compound has already deposited in the usual manner, and the novel mixture then charged to the reactor at start up of the process at the oxyhydrochlorination plant. Further, the bare support can be added during operation of the process at a time when the fluid bed is composed solely of supported catalyst or thereafter, at intervals as required to provide makeup catalyst or to alleviate stickiness developing in the bed. It is also possible to eliminate preparation of the catalyst outside the reactor by charging the reactor with bare fluidizable support, passing reactant gases through the reactor to fluidize the bed at the reaction temperature and then adding copper compound, preferably copper chloride, to the reactor. In this manner, the entire amount of supported catalyst is prepared in situ. It is generally preferred, however, to form only a portion of the supported catalyst in situ, the other portion being prepared in the usual manner outside the reactor.

When bare fluidizable support is added to the oxyhydrochlorination reactor and mixed with already supported copper catalyst, subsequent operation of the reactor results in a rapid release of a portion of the copper compound from the supported catalyst, particularly that which is concentrated on the outside surfaces of the support particle, and the copper, in situ, deposits on or transfers to the particles of the bare support. This is evidenced by a change in color of the mass of the fluidized bed from the speckled appearance characteristic of a mixture of greenish copper bearing particles and white bare support particles, to a uniform pale greenish color throughout. It is believed that the fluidized condition of the bare support particles under reaction conditions unexpectedly permits the copper compound to permeate their pores and be distributed throughout their mass in a more efficient and uniform manner than is possible when the copper compound is placed on the support in the usual manner outside the reactor.

In any event, operation of the fluid bed to effect the oxyhydrochlorination reaction after deposit of copper compound on bare support particles in situ, whether or not all or only a substantial portion of the support particles have received their deposit of copper compound while fluidized, inhibits or reduces the tendency of the catalyst to develop stickiness in the fluidized bed which may otherwise be encountered, and fluidization characteristics of the bed are substantially improved.

From the foregoing, this invention, in one aspect, constitutes a new and improved method or process for conducting oxyhydrochlorination reactions, particularly the reaction of ethylene, oxygen and HCl to produce EDC, in a fluidized bed of copper supported catalyst characterized in that at least a portion of the copper is deposited in situ on at least a portion of the fluidizable support. In another aspect, the invention constitutes a novel composition of matter, i.e. a mixture of copper supported catalyst with bare support, adapted to be used in and to improve the operation of fluid bed oxyhydrochlorination reactions.

Detailed description of invention including specific embodiments

In the practice of the process of this invention, according to one preferred embodiment, the oxyhydrochlorination of ethylene to produce EDC is begun in the manner described in the referenced Harpring et al patent using a fluid bed composed of copper compound supported on alumina which is prepared outside the reactor in the known manner. Bare alumina support is added to the fluid bed when there is indication that the catalyst is developing stickiness or losing its ability to fluidize, and the oxyhydrochlorination reaction is then continued whereupon copper from the already supported catalyst deposits on the bare support in situ and the stickiness is alleviated.

In another preferred embodiment of the process a mixture of bare alumina support and copper catalyst already supported on alumina is prepared by intimately mixing the two outside the reactor and then adding the mixture to the reactor. In this case, the oxyhydrochlorination of ethylene to produce EDC is effected in a fluid bed initially composed of the mixture, whereby development of stickiness during the reaction is inhibited as a portion of the copper from the supported copper catalyst deposits itself in situ on the bare support in the fluid bed as the reaction proceeds.

The bare support alone or the mixture described may also, in still other embodiments of the invention, be supplied to the operator of the oxyhydrochlorination process and used as make up when the catalyst bed needs replenishing. Here too, a portion of the copper is deposited on the bare support in situ and stickiness in the catalyst bed is alleviated.

The composition of this invention is the mixture of bare alumina support and the supported copper catalyst.

In each of these embodiments of the invention, it is desirable but not essential that the bare alumina support used be the same alumina as that on which the copper catalyst is deposited to produce the supported catalyst.

The preferred support used as bare support or as the support on which the copper compound is initially deposited is an alumina support and can be any of the fluidizable aluminas well known to the art including gamma-alumina, alpha-alumina, the so-called "Condea" alumina, microgel alumina, "activated" alumina, and the like. It may also be a modified alumina support in which is incorporated prior to deposit of copper, from 0.5 to 1.5% of an alkali-alkaline earth or rare earth metal. The particle size of the alumina support is preferably such that 95 to 90 weight percent of the particles are below 80 microns in diameter, 40 to 50% below 45 microns in diameter and 15 to 30% below 30 microns in diameter with no more than 3 to 10% by weight of the particles smaller than 20 microns in diameter and no more than 1 to 5% larger than 200 microns in diameter. The bulk density of the preferred alumina supports is in the range of 0.8 to 1.1 grams per cc and their surface area is in the range of 60 to 150 sq. meters per gram.

Such bare alumina supports are white in color and except for a change to a greenish color, and other changes in physical characteristics as a result of copper deposits, including a 10 to 30 percent reduction in surface area, the above description of bare support is also descriptive of the copper catalyst supported on alumina with which the bare support is mixed or to which the bare support is added in the fluid bed.

The amount of bare alumina support admixed with supported copper catalyst, either prior to or after addition to the reactor, may be varied depending on the copper content of the initial supported catalyst and the desired copper content of the final supported catalyst. To achieve a significant change in the character of the fluid bed, a minimum of 5% of the weight of the entire bed should be added as bare support up to a maximum of 50%, it being understood, however, that the limit on the maximum use of bare support is such that the final copper content, which is obviously diluted by use of bare support, is not reduced below 2%. It should be noted, however, that the inhibition or amelioration of stickiness and improved performance of the catalyst bed effected by this invention is not due solely to reduced copper content. As will be exemplified below, a supported catalyst of a given copper content prepared in part by depositing copper on support in situ, as described, is less sticky than a catalyst of the same copper content prepared by depositing all the copper on the support before addition to the reactor.

The method and composition of this invention are particularly useful to improve the ethylene oxyhydrochlorination process when carried out at a temperature of 235°C or more using a high copper content catalyst wherein the copper is supported on an alumina support having a surface area in the range of 60 to 80 square meters per gram. Operation of the process under these conditions without taking advantage of this invention can often lead to an undesirable degree of stickiness of the catalyst bed and can lead to loss of catalyst and/or hot spots in the fluid bed.

To demonstrate the extent to which use of bare support in accordance with this invention produces the desired results, it is desirable that a test method be available for determining the degree of stickiness of a given catalyst at commercial operating conditions. A test has been devised for this purpose called the Temperature Profile Test (TPT). In this test a bench-scale fluid bed reactor of 30 mm internal diameter is equipped with means for pre-heating and delivering a mixture of ethylene, air and HCl through a mass of supported catalyst of a given height contained in the reactor. A thermocouple is used to measure the temperature of the fluid bed at each point in its height by gradually raising the thermocouple through the height of the bed. The laboratory reactor is operated under a given sequence

of operating conditions and the temperature of the bed throughout its height is noted for each specific operating condition. If the temperature varies from one point in the bed to another, for any given condition, this is an indication of stickiness and a rating, indicating stickiness, can be assigned to this supported catalyst being tested. The stickiness varies from a rating of 1 which indicates no temperature variation in any of the specific operating conditions, and hence, no stickiness, to a rating of 4 which indicates temperature variations throughout the height of the bed under each set of operating conditions and extreme stickiness. During the test, in each sequence of operating conditions, the conversion of ethylene to EDC and the yield and efficiency of EDC production can be measured so as to ascertain the effectiveness of a given supported catalyst with a determined stickiness rating in EDC production.

There is a sophisticated method and apparatus for determining the precise consistency and stickiness of an operating fluid bed and for selecting the most desirable supported catalyst among a number of catalysts (and hence, demonstrating that the supported catalyst of this invention as prepared by depositing a portion of copper on the support in situ are less sensitive and sticky than those prepared entirely outside the reactor) which is described in the copending application of Joseph A. Cowfer, et al, Serial No. 949,170 filed October 6, 1978, the disclosure of which is incorporated herein by reference. As described therein in detail, the apparatus used is a laboratory fluid bed provided with a pendulum viscometer which measures the rate of damping or k value of a predetermined torsional oscillation of the pendulum during operation of the fluid bed with any given supported catalyst, and the k values as obtained for different supported catalyst can be compared to select the catalyst which is least prone to produce stickiness during the oxhydrochlorination reaction.

The method and composition of this invention and the advantages achieved thereby, are further illustrated in the following specific examples.

Example I

An alumina supported copper catalyst is prepared by dissolving 26.84 g of $CuCl_2 \cdot 2H_2O$ in 110 ml water and adding the solution in portion to 95 g of fluidizable gamma-alumina powder of the following range of particle sizes by weight: 6% below 20 microns; 15—28% less than 30 microns, by 40—50% less than 45 microns and 75—91% less than 80 microns, and having a surface area of 70 to 100 square meters per gram. The wetted alumina is evaporated to dryness on a steam bath (80°C) until it becomes free flowing, calcined for 16 hours at 110°C and the dry finely divided supported catalyst screened through a 20 mesh screen. The supported catalyst thus prepared is of the consistency of finely divided sand and has a greenish color. Its copper content is 10% by weight. Its surface area is less than that of the alumina used as the support due to deposit of copper on the surfaces of the particles and is in the range of 60 to 85 square meters per gram.

A portion of this alumina supported copper catalyst is mixed thoroughly by stirring with an equal amount by weight of the same alumina support used in preparing the supported catalyst. The white particles of bare support and the greenish particles of supported catalyst are evidenced in the mixture. The percentage of copper in the mixture is now 5% by weight.

The supported catalyst and the composition of this invention, i.e. the mixture of supported catalyst with bare support, is each separately tested in the TPT for stickiness when used as the fluid bed catalyst in the reaction of ethylene, oxygen and HCl to produce EDC. In each case, 125 l of fluid bed material is placed in the 30 mm internal diameter fluid bed reactor to produce a bed height of 12 inches and the reactor operated by passing gaseous ethylene, oxygen (supplied as air) and HCl in a ratio of ethylene to oxygen to HCl of 1/0.8/2.0 through the bed while varying the temperature of the reaction in the fluid bed and the contact time of the gaseous reactants in the bed. In each case, any variations in temperature through the height of the bed, as well as the conversion of ethylene and the yield of EDC are measured.

Using the supported catalyst alone, at a temperature of 219°C and a contact time of 13.1 seconds, a 66.2% conversion of ethylene is secured with 66% ethylene to EDC efficiency (yield of EDC times ethylene conversion) without variation in the 219°C temperature throughout the height of the bed. However, at higher temperatures in the range of 221 to 235°C with contact time in the range of 14 to 22 seconds, when using the supported catalyst alone, the temperature at the bottom of the bed increased by several degrees. The catalyst became undesirably sticky as to prevent proper fluidization in the bed. This catalyst had a TPT stickiness rating of 4.

In contrast, under the same conditions, the TPT results using the equal mixture of supported catalyst and bare support shows no change in temperature throughout the height of the bed during the tests. Operating conditions used temperatures varying from 221 to 235°C and contact times varying from 14 to 22 seconds. The ethylene conversions range from 78.4 to 97.8% and the ethylene efficiencies to EDC from 78.6 to 91.9%. This example illustrates that stickiness is inhibited. Furthermore, in this example, improved EDC efficiency was obtained by the addition of bare support to the already supported copper catalyst.

Example II

Using the materials and procedures and conditions of Example I, alumina supported copper

chloride catalyst is prepared to contain 5% by weight of copper. This catalyst is tested by the TPT, and a stickiness rating of 3.5 is obtained. The same catalyst is then mixed in equal proportions with the same bare alumina support used in the above preparation and the mixture tested in the TPT which gives a TPT stickiness rating of 1. This mixture, containing 2.5% copper, was then used as the initial fluid bed in a laboratory fluid bed ethylene oxyhydrochlorination reactor of 30 MM internal diameter with a catalyst bed height of 15 inches in a run of 85 hours duration. No stickiness is observed in the entire period of observation and the bed fluidizes excellently throughout. Data for this run is shown in Table I.

TABLE I

| Temp. 0°C | Contact time Seconds | Hours Elapsed | Reactant ratios $C_2H_4$ | $O_2$ | HCl | % Yield CO | $CO_2$ | EDC | % Efficiency EDC |
|---|---|---|---|---|---|---|---|---|---|
| 227 | 14.4 | 3 | 1 | 0.78 | 1.86 | 2.01 | 1.88 | 95.6 | 78.5 |
| 223 | 14.5 | 9 | 1 | 0.78 | 1.86 | 1.34 | 1.45 | 96.9 | 91.8 |
| 221 | 19.7 | 11 | 1 | 0.78 | 1.86 | 1.15 | 1.31 | 97.0 | 94.6 |
| 228 | 19.4 | 13 | 1 | 0.78 | 1.86 | 1.56 | 1.65 | 96.1 | 94.7 |
| 228 | 19.4 | 36 | 1 | 0.78 | 1.86 | 1.83 | 1.69 | 95.8 | 94.9 |
| 229 | 24.8 | 38 | 1 | 0.8 | 1.96 | 2.35 | 2.39 | 94.2 | 94.2 |
| 231 | 20.5 | 56 | 1 | 0.8 | 2.02 | 2.20 | 2.40 | 94.7 | 93.5 |
| 234 | 21.7 | 61 | 1 | 0.8 | 2.23 | 1.98 | 1.99 | 95.1 | 94.7 |
| 235 | 21 | 62 | 1 | 0.8 | 2.12 | 2.04 | 1.98 | 95.2 | 95.2 |
| 238 | 21 | 81 | 1 | 0.8 | 2.12 | 2.55 | 2.71 | 93.7 | 93.7 |
| 236 | 20 | 85 | 1 | 0.7 | 2.0 | 2.24 | 2.33 | 94.6 | 93.3 |

Catalyst mixture rated #1 in TPT
Supported catalyst is rated #3.75 in TPT

Example III

The materials and procedures and conditions of Example I are again repeated using various lots of alumina supported copper catalyst with various ratios of copper and various proportions of bare support. The results are shown in Table II. In the column headed "Reactor Fluid Bed Condition" the designation "sticky" corresponds to a TPT stickiness rating of 3 to 4, and the designation "fluid" corresponds to a TPT stickiness rating of 1.

TABLE II

| Supported catalyst Lot | Weight | % Cu. | Weight bare support | % Of copper mixture | Reactor fluid bed condition |
|---|---|---|---|---|---|
| A | 123 | 3.9 | 0 | — | Sticky |
| A | 123 | 3.9 | 7.2 | 3.7 | Fluid |
| B | 124 | 5.0 | 0 | — | Sticky |
| B | 74.5 | 5.0 | 74.5 | 2.5 | Fluid |
| C | 116 | 5.0 | 0 | — | Sticky |
| C | 85 | 5.0 | 56.7 | 3.0 | Fluid |
| C | 65 | 5.0 | 65 | 2.5 | Fluid |

Example IV

In this Example, a copper on alumina supported catalyst is prepared as described in Example I entirely outside the reactor. The catalyst contains 3.75% copper and is tested in the TPT procedure. It has a rating of 3.0. In contrast, a mixture of copper on alumina catalyst containing 5% copper and bare alumina is prepared to give an overall copper content of 3.75%. This mixture is similarly tested and found to have a rating of 1 in the TPT procedure. This Example clearly indicates that the inhibition of stickiness is not due solely to a reduction in overall copper content, and that the deposit of a portion of the copper on the bare support is advantageous in inhibiting the development of stickiness in the fluid bed.

Example V

This Example illustrates the preparation of fluid bed copper-on-alumina catalyst entirely inside the reactor bed by depositing copper chloride on bare support in situ. The bare alumina support described in the previous Example is placed in the reactor zone of the laboratory scale fluid bed reactor described in Example II and a gaseous mixture of 1 mole ethylene and 0.8 mole oxygen for each 2 moles of HCl is passed therethrough with the reactants at a temperature of 235°C. In one run, of 12 hours duration, solid cupric chloride is charged at the beginning of the run and the reactor, while maintaining reaction conditions, in an amount sufficient to give a copper concentration on the support of 1.6% and in

another run of 110 hours duration the amount of cupric chloride at the beginning of the run is sufficient to give a copper concentration on the support of 2.8%. In the first run the conversion of ethylene is 52.9% and the second run, with a larger copper concentration, the maximum ethylene conversion is 81%. For both cases EDC yield from converted ethylene is 94—95%. Catalyst fluidization is not affected by addition of the solid cupric chloride and the fluid bed does not develop "stickiness" during either run.

**Claims**

1. The process of oxyhydrochlorinating a gaseous hydrocarbon containing 1 to 4 carbon atoms to form a chlorinated hydrocarbon, in which the hydrocarbon, an oxygen containing gas and gaseous hydrogen chloride are passed at a temperature of 190° to 250°C through a fluidized bed of a catalyst containing a copper compound deposited on a fluidizable support, characterized in that at least a substantial portion of the copper compound is deposited on at least a substantial portion of the fluidizable support in situ in the fluidized bed, thereby preventing particles of supported catalyst from sticking to one another in the fluid bed and adversely affecting its fluidization properties.

2. The process of Claim 1 wherein the hydrocarbon is ethylene and 1,2-dichloroethane is produced.

3. The process of Claim 2 wherein the copper compound is cupric chloride and the fluidizable support on which it is deposited is alumina having a surface area within the range of 60 to 160 square meters per gram.

4. In a process of producing 1,2-dichloroethane by reacting a gaseous mixture of ethylene, oxygen and hydrogen chloride in a ratio of 1.0 to 1.2 moles ethylene and 0.5 to 0.9 moles oxygen for each 2 moles of HCl at a temperature in the range of 190° to 250°C in a fluidized bed of a solid catalyst of cupric chloride supported on alumina, containing from 2% to 10% copper and prepared by deposit of the cupric chloride on the bare support prior to its use in the fluidized bed reaction, the improvement which comprises adding to the fluidized bed bare alumina support in an amount sufficient that it constitutes from 5% to 50% by weight of the catalyst solids in the bed and continuing the reaction whereby a portion of the cupric chloride on the supported catalyst in the fluid bed is deposited in situ on the particles of bare alumina support, and stickiness of the supported catalyst is substantially alleviated.

5. The process of Claim 4 wherein the molar ratio of ethylene to oxygen to hydrogen chloride is 1.0 to .08 to 2.0, the reaction is conducted at a temperature of 235°C and the alumina used has a surface area within the range of 60 to 160 square meters per gram.

6. The method of producing 1,2-dichloroethane which comprises the steps of (A) preparing a mixture of (1) a supported catalyst consisting of a copper compound deposited on a bare alumina support and (2) bare alumina support on which no copper is deposited, the proportion of (2) being from 5% to 50% by weight of the total mixture, (B) fluidizing the mixture produced in step (A) to produce a fluidized bed of solid particles, and (C) passing a gaseous mixture of ethylene, and oxygen containing gas and hydrogen chloride through the fluidized bed while maintaining its temperature in the range of 190° to 250°C, whereby copper compound from (1) is deposited on (2) in situ and stickiness in the fluidized bed is substantially alleviated while the ethylene is converted to 1,2-dichloroethane.

7. The method of Claim 6 wherein the step (A) component (1) of the mixture is a supported catalyst consisting of copper chloride deposited on finely divided alumina having a surface area of 60 to 160 square meters per gram, component (2) is the same finely divided alumina as that used in the support in (1) and in step (C) the molar ratio of ethylene to oxygen to hydrogen chloride is 1.0 to 0.8 to 2.0 and the temperature is maintained at 235 to 240°C.

8. A composition of matter adapted for use as a catalyst in the fluid bed oxyhydrochlorination of 1 to 4 carbon atom hydrocarbons to produce chlorinated hydrocarbons consisting essentially of a mixture of (1) copper compound deposited on fluidizable support and containing 2 to 10% by weight of copper and (2) bare fluidizable support, the proportion of (2) being from 5 to 50% by weight of the total mixture of (1) and (2).

9. A composition of Claim 8 wherein the 1 to 4 carbon atom hydrocarbon is ethylene and the product made is essentially 1,2-dichloroethane.

10. A composition of Claim 8 wherein the fluidizable support is an alumina support, and the copper compound is cupric chloride.

**Patentansprüche**

1. Verfahren zur Oxyhydrochlorierung eines gasförmigen Kohlenwasserstoffs mit 1 bis 4 Kohlenstoff-Atomen zu einem gasförmigen chlorierten Kohlenwasserstoff, in dem der Kohlenwasserstoff, ein Sauerstoff enthaltendes Gas und gasförmiges Hydrogenchlorid bei einer Temperatur von 190°C bis 250°C durch ein Fließbett eines Katalysators geleitet werden, der eine Kupfer-Verbindung aufgelagert auf einem fluidisierbaren Träger enthält, dadurch gekennzeichnet, daß wenigstens ein wesentlicher Teil der Kupfer-Verbindung auf wenigstens einen wesentlichen Teil des fluidisierbaren Trägers in situ in dem Fließbett aufgelagert wird, wodurch verhindert wird, daß die Teilchen des

Trägerkatalysators in dem Fließbett aneinander kleben und dessen Fluidisationseigenschaften nachteilig beeinflussen.

2. Verfahren nach Anspruch 1, dadurch gekennzeichnet, daß der Kohlenwasserstoff Ethylene ist und 1,2-Dichloroethan erzeugt wird.

3. Verfahren nach Anspruch 2, dadurch gekennzeichnet, daß die Kupfer-Verbindung Kupfer(II)-chlorid ist und der fluidisierbare Träger, auf den es aufgelagert wird, Aluminiumoxid mit einer spezifischen Oberfläche von 60 bis 160 $m^2/g$ ist.

4. Verfahren zur Herstellung von 1,2-Dichloroethan durch Reaktion einer gasförmigen Mischung von Ethylen, Sauerstoff und Hydrogenchlorid in einem Verhältnis von 1,0 bis 1,2 mol Ethylen und 0,5 bis 0,9 mol Sauerstoff zu jeweils 2 mol HCl bei einer Temperatur im Bereich von 190°C bis 250°C in einem Fließbett eines festen Katalysators von Kupfer(II)-chlorid auf einem Aluminiumoxid-Träger, wobei der Katalysator 2 bis 10% Kupfer enthält und vor seiner Verwendung durch Auflagerung des Kupfer(II)-chlorides auf dem bloben Träger in dem Fließbett hergestellt wurde, dadurch gekennzeichnet, daß dem Fließbett der bloße Aluminiumoxid-Träger in hinreichender Menge zugesetzt wird, so daß sie 5 bis 50% Gew.-% der Katalysator-Feststoffe in dem Bett ausmacht, und die Reaktion fortgeführt wird, wobei ein Teil des Kupfer(II)-chlorids auf dem Trägerkatalysator in dem Fließbett in situ auf die Teilchen des bloben Aluminiumoxid-Trägers aufgelagert wird und ein Kleben des Trägerkatalysators beträchtlich vermindert wird.

5. Verfahren nach Anspruch 4, dadurch gekennzeichnet, daß das Stoffmengenverhältnis ("Molverhältnis") Ethylen zu Sauerstoff zu Hydrogenchlorid 1,0:0,8:2,0 ist, die Reaktion bei einer Temperatur von 235°C durchgeführt wird und das Aluminiumoxid eine spezifische Oberfläche im Bereich von 60 bis 160 $m^2/g$ besitzt.

6. Verfahren zur Herstellung von 1,2-Dichloroethan, umfassend die Schritte des

(A) Herstellens einer Mischung aus

(1) einem aus einer Kupfer-Verbindung, die auf einem bloßen Aluminiumoxid-Träger aufgelagert ist, bestehenden Trägerkatalysator und

(2) einem bloßen Aluminiumoxid-Träger, afu dem kein Kupfer aufgelagert ist, wobei der Anteil von (B) in der Mischung von 5 bis 50 Gew.-%, bezogen auf das Gesamtgewicht der Mischung, beträgt,

(B) Fluidisierens der in Schritt (A) erzeugten Mischung zur Herstellung eines Fließbettes aus festen Teilchen, und

(C) Hindurchleitens einer gasförmigen Mischung aus Ethylen, einem Sauerstoff enthaltenden Gas und Hydrogenchlorid durch das Fließbett, während dessen Temperatur auf 190°C bis 250°C gehalten wird, wobei Kupfer-Verbindung aus (1) auf (2) in situ aufgelagert wird und ein Kleben in dem Fließbett beträchtlich vermindert wird, während das Ethylen in 1.2-Dichloroethan überführt wird.

7. Verfahren nach Anspruch 6, dadurch gekennzeichnet, daß in Schritt (A) die Komponente (1) ein Trägerkatalysator, bestehend aus Kupferchlorid aufgelagert auf einem feinteiligen Aluminiumoxid mit einer spezifischen Oberfläche von 60 bis 160 $m^2/g$, ist, die Komponente (2) das gleiche feinteilige Aluminiumoxid ist, wie es in der Komponente (1) als Träger verwendet wurde, und in Schritt (C) das Stoffmengenverhältnis Ethylen zu Sauerstoff zu Hydrogenchlorid 1,0:0,8:2,0 ist und die Temperatur bei 235°C bis 240°C gehalten wird.

8. Stoffmischung zur Verwendung als Katalysator in der Fließbett-Oxyhydrochlorierung von Kohlenwasserstoffen mit 1 bis 4 Kohlenstoff-Atomen zur Erzeugung gasförmiger chlorierter Kohlenwasserstoffe, bestehend im wesentlichen aus einer Mischung aus (1) einer Kupfer-Verbindung aufgelagert auf einem fluidisierbaren Träger und enthaltend 2 bis 10 Gew.-% Kupfer, und (2) dem bloßen fluidisierbaren Träger, wobei der Anteil von (2) 5 bis 50 Gew.-%, bezogen auf das Gesamtgewicht von (1) und (2), beträgt.

9. Stoffmischung nach Anspruch 8, dadurch gekennzeichnet, daß der Kohlenwasserstoff mit 1 bis 4 Kohlenstoff-Atomen Ethylen ist und das hergestellte Produkt im wesentlichen 1,2-Dichloroethan ist.

10. Stoffmischung nach Anspruch 8, dadurch gekennzeichnet, daß der fluidisierbare Träger ein Aluminiumoxid-Träger ist und die Kupfer-Verbindung Kupfer(II)-chlorid ist.

**Revendications**

1. Le procédé d'oxyhydrochloration d'un hydrocarbure gazeux renfermant 1 à 4 atomes de carbone pour former un hydrocarbure chloré, dans lequel on fait passer l'hydrocarbure, un gaz contenant de l'oxygène et du chlorure d'hydrogène gazeux à une température de 190 à 250°C à travers un lit fluidisé d'un catalyseur contenant un dérivé du cuivre déposé sur un support fluidisable, caractérisé en ce qu'au moins une fraction substantielle du dérivé du cuivre est déposée sur au moins une partie substantielle du support fluidisable in situ dans le lit fluidisé, empêchant ainsi les particules de catalyseurs fixées au support de coller les unes aux autres dans le lit fluidisé et d'influer défavorablement sur ses propriétés de fluidisation.

2. Procédé selon la revendication 1, caractérisé en ce que l'hydrocarbure est l'éthylène et que du 1,2-dichloroéthane est formé.

3. Procédé selon la revendication 2, caractérisé en ce que le dérivé du cuivre est le chlorure cuivrique et, que le support fluidisable sur lequel il est déposé est de l'alumine possédant une aire spécifique comprise entre 60 et 160 $m^2$ par gramme.

4. Procédé de fabrication de 1,2-dichloroéthane par réaction d'un mélange gazeux d'éthylène, d'oxygène et de chlorure d'hydrogène dans une rapport de 1 à 1,2 moles d'éthylène et de 0,5 à 0,9 mole d'oxygène pour 2 moles d'HCl à une température comprise entre 190 et 250°C dans un lit fluidisé d'un catalyseur solide de chlorure cuivrique sur support d'alumine contenant 2% à 10% de cuivre et préparé par dépôt du chlorure cuivrique sur le support nu avant son utilisation dans la réaction en lit fluidisé, caractérisé en ce que l'on ajoute au lit fluidisé un support d'alumine nu en une quantité suffisante pour qu'il constitue 5% à 50% en poids des solides catalyseurs dans le lit et que l'on poursuit la réaction, grâce à quoi une partie du chlorure cuivrique du catalyseur sur support dans le lit fluide se dédépose in situ sur les particules de support d'alumine nu et le pouvoir d'adhésion du catalyseur sur support est sensiblement atténué.

5. Procédé selon la revendication 4, caractérisé en ce que le rapport molaire d'éthylène à l'oxygène au chlorure d'hydrogène est de 1/0 à 0,8 à 2,0, que la réaction est effectuée à une température de 235°C et que l'alumine utilisée possède une aire spécifique comprise entre 60 à 160 $m^2$ par gramme.

6. Le procédé de préparation de 1,2-dichloroéthane qui comprend les étapes (A) de préparation d'un mélange d'un catalyseur sur support (1) consistant en un dérivé du cuivre déposé sur un support d'alumine nu et d'un support d'alumine nu (2) sur lequel n'est pas déposé de cuivre, la proportion de (2) étant de 5% à 50% en poids du mélange total, (B) de fluidisation du mélange produit dans l'étape (A) pour formerun lit fluidisé de particules solides et (C) de passage d'un mélange gazeux d'éthylène, d'un gaz contenant de l'oxygène et de chlorure d'hydrogène à travers le lit fluidisé tout en maintenant sa température entre 190 et 250°C, moyennant quoi le dérivé du cuivre provenant de (1) est déposé in situ sur (2) et l'adhésion dans le lit fluidisé est sensiblement atténué pendant qu l'éthylène est converti en 1,2-dichloroéthane.

7. Procédé selon la revendication 6, caractérisé en ce que le constituant (1) du mélange de l'étape (A) est un catalyseur sur support consistant en du chlorure de cuivre déposé sur de l'alumine finement divisée ayant une aire spécifique de 60 à 160 $m^2$ par gramme, que le constituant (2) est la même alumine finement divisée que celle utilisée dans le support de (1) et que dans l'étape (C) le rapport molaire d'éthylène à l'oxygène au chlorure d'hydrogène est de 1 à 0,8 à 2,0 et la température est maintenue entre 235 et 240°C.

8. Composition destinée à l'emploi comme catalyseur dans l'oxyhydrochloration en lit fluidisé d'hydrocarbures ayant 1 à 4 atomes de carbone pour produire des hydrocarbures chlorés consistant essentiellement en un mélange d'und érivé du cuivre (1) déposé sur un support fluidisable et contenant 2 à 10% en poids de cuivre et d'un support fluidisable nu (2), la proportion de (2) étant de 5 à 50% en poids du mélange total de (1) et (2).

9. Composition selon la revendication 8, caractérisée en ce que l'hydrocarbure ayant 1 à 4 atomes de carbone est l'éthylène et que le produit obtenu est essentiellement du 1,2-dichloroéthane.

10. Composition selon la revendication 8, caractérisée en ce que le support fluidisable est un support d'alumine et que le dérivé du cuivre est du chlorure cuivrique.